(19) Europäisches Patentamt
European Patent Office
Office européen des brevets

(11) Numéro de publication: **0 243 248**
**A1**

(12) # DEMANDE DE BREVET EUROPEEN

(21) Numéro de dépôt: **87400868.3**

(51) Int. Cl.⁴: **A 61 K 7/06**

(22) Date de dépôt: **15.04.87**

(30) Priorité: **18.04.86 LU 86396**

(43) Date de publication de la demande:
**28.10.87 Bulletin 87/44**

(84) Etats contractants désignés:
**AT BE CH DE ES FR GB GR IT LI NL SE**

(71) Demandeur: **LABORATOIRES PHARMACEUTIQUES ROCHE-POSAY**
**Chemin du Casino**
**F-86270 Roche-Posay (FR)**

**Venencie, Pierre-Yves**
**2 rue Saint James**
**F-92200 Neuilly sur Seine (FR)**

(72) Inventeur: **Venencie, Pierre-Yves**
**2 rue Saint James**
**F-92200 Neuilly sur Seine (FR)**

**Levayer, René**
**46 rue Paul Heluard**
**F-76000 Le Havre (FR)**

(74) Mandataire: **Phélip, Bruno et al**
**c/o Cabinet Harié & Phélip 21, rue de la Rochefoucauld**
**F-75009 Paris (FR)**

(54) **Compositions pour le traitement de la calvitie et des alopécies.**

(57) Compositions cosmétiques et pharmaceutiques présentées en vue de l'application topique et contenant, à titre d'agent actif, l'un au moins des produits connus sous les Dénominations Communes Internationales (DCI) Phénytoin (ou Phénytoïne) et Diazoxide (ou Diazoxyde), ainsi que leurs sels acceptables du point de vue cosmétique ou pharmaceutique.

Application au traitement préventif ou curatif de la calvitie et des alopécies.

EP 0 243 248 A1

**Description**

La présente invention concerne le domaine du traitement préventif ou curatif de la calvitie et des alopécies.

Elle a en particulier pour objet de nouvelles compositions destinées à prévenir la chute des cheveux, voire même à favoriser leur repousse.

Les compositions selon l'invention sont également efficaces dans le traitement des alopécies diffuses, en particulier les alopécies androngéniques, les alopécies témoins d'une pathologie générale (hypothyroidie, insuffisance antihypophysaire, diabète sucré, anémie hypochrome, lupus érythémateux systémique, carences alimentaires ....), les alopécies de stress (accouchement , intervention chirurgicale, choc psycho-affectif) et autres affections du même type .

Les alopécies androgéniques dites séborrhéiques sont considérées comme responsables de la calvitie commune . Elles atteignent fréquemment l'homme entre 25 et 40 ans. Elles débutent par un éclaircissement des golfes temporaux et évoluent en quelques années vers l'alopécie hypocratique .

Beaucoup plus rares chez la femme, elles présentent un aspect diffus avec absence de zones complètement chauves. La séborrhée est généralement importante .

L'invention a pour objet des compositions cosmétiques et pharmaceutiques présentées en vue de l'application topique et contenant, à titre d'agent actif, l'un au moins des produits connus sous les Dénominations Communes Internationales (DCI) Phenytoïn (ou Phénytoïne) et Diazoxide (ou Diazoxyde) , ainsi que leurs sels acceptables du point de vue cosmétique ou pharmaceutique.

Le Diazoxyde (voir The Merck Index 9ème 2dition, 1976, page 395, no 2968) est un composé correspondant à la formule suivante:

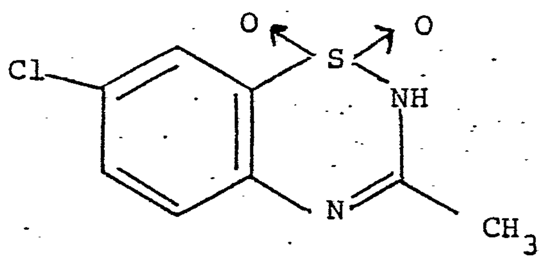

de nomenclature chloro-7 méthyl-3 benzothiadiazine 1, 2,4 dioxyde-1,1.

Ce composé de formule brute $C_8 H_7 Cl N_2 O_2 S$ (PM: 230,70) se présente sous forme de cristaux, de point de fusion voisin de 331°C, insolubles dans l'eau, solubles dans l'alcool et les solutions alcalines . Le diazoxyde a déjà été proposé comme médicament antihypertenseur.

La Phenytoïne (voir The Merck Index 9ème Edition, 1976, page 952, no 7130), également appelée Diphénylhydantoïne, est un composé correspondant à la formule :

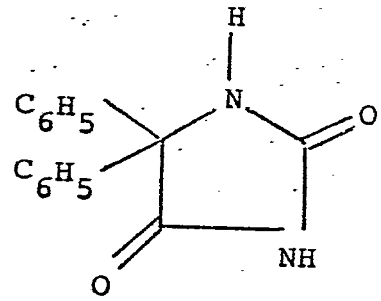

de nomenclature diphényl-5,5 imidazolidinedione-2,4 . La phénytoïne a déjà été proposée comme médicament anticonvulsivant et antiépileptique.

Ce composé de formule brute $C_{15} H_{12} N_2 O_2$ ( PM : 252,26) se présente sous la forme de poudre de point de fusion compris entre 295 et 298°C , pratiquement insoluble dans l'eau, soluble dans l'alcool (1g/60 ml) et dans l'acétone (1 g/30 ml).

L'invention concerne également les sels des produits précités, qui conviennent à l'application locale. On connaît notamment les sels alcalins et alcalino-terreux de la phénytoïne , en particulier les sels de sodium et de calcium . A titre illustratif, le diphénylhydantoïnate de sodium est un composé correspondant à la formule :

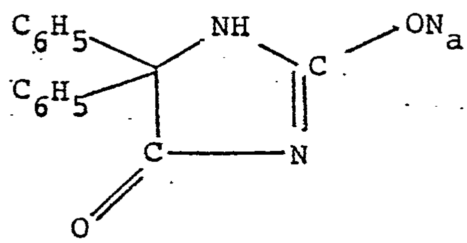

Ce sel se présente sous forme d'une poudre blanche, légèrement hygroscopique un peu soluble à l'eau, l'alcool, soluble en milieu alcalin, insoluble dans l'ether, le chloroforme.

Les nouvelles compositions sont adaptées à l'application topique sur le cuir chevelu. A côté du ou des agents actifs (phénytoïne et/ou diazoxyde) , la composition contient un excipient qui peut être choisi par l'homme du métier parmi les excipients et véhicules traditionnels pour ce type d'application. Ainsi, selon l'invention, la composition peut se présenter sous différentes formes, notamment sous forme de lotion, de gel, d'émulsion, de pommade.

Le terme "pommade" couvre des formulations comprenant des bases oléagineuses absorbables, par exemple la vaseline, la lanoline, les polyéthylèneglycols ainsi que leurs dérivés et leurs mélanges.

Ces pommades peuvent être préparées par dispersion du diazoxyde, de la phénytoïne ou de leurs sels ou par solubilisation desdits produits à l'aide d'un tiers solvant.

Les émulsions, qu'elles soient du type huile dans eau ou eau dans huile, sont préparées en dispersant le diazoxyde dans la phase aqueuse et la phénytoïne

est dispersée en fonction de son affinité, soit dans la phase grasse soit dans la phase aqueuse avant la mise en émulsion. En variante, on peut également solubiliser les agents actifs à l'aide d'un tiers solvant.

Le rapport en poids de la phase grasse à la phase aqueuse est généralement compris entre 95/5 et 25/75 .

Parmi les différentes huiles susceptibles de constituer la phase grasse, on peut utiliser divers produits, tels que:

-des huiles animales comme la lanoline, le perhydrosqualène ,

-des huiles végétales comme l'huile d'amande douce, l'huile d'avocat, l'huile de ricin, l'huile d'olive, l'huile de pépins de raisin, l'huile d'oeillette, l'huile de colza, l'huile d'arachide, l'huile de maïs, l'huile de noisette, l'huile de jojoba, l'huile de carthame, l'huile de germes de blé, le beurre de karité et la graisse de Shorea robusta,

-des huiles minérales comme par exemple l'huile de paraffine,

-des huiles de silicone solubles dans les autres huiles.

On peut également utiliser certains produits synthétiques tels que par exemple, des esters saturés et notamment le palmitate d'isopropyle, le myristate d'isopropyle, de butyle et de cétyle , le stéarate d'hexadécyle, le palmitate d'éthyle ainsi que des triglycérides des acides octanoïque et décanoïque et le ricinoléate de cétyle, l'huile de purcellin et le polyisobutène hydrogéné.

La phase grasse des émulsions peut également contenir certaines cires et notamment de la cire de carnauba, de la cire d'abeille, de l'ozokérite ou de la cire de candellila.

Les lotions sont des solutions ou des dispersions de diazoxyde qui lui est associé dans un support approprié . Comme support approprié, on peut citer les alcools tels que l'éthanol, les polyols tels que la glycérine, les(poly)alkylèneglycols, les solvants organiques compatibles avec la peau, ainsi que l'eau et les mélanges de ceux-ci.

Les gels sont des préparations semi-solides obtenues par gélification d'une solution ou d'une suspension de diazoxyde, à l'aide d'agents gélifiants tels que les montmorillonites organophilées (Bentone),pour une phase grasse, où l'acide polyacrylique réticulé pour une phase aqueuse , les dérivés de cellulose, les gommes d'origine naturelle ou synthétique.

On peut si on le désire, introduire dans le gel un tensio-actif, ce qui permet une meilleure dispersion et disponibilité du diazoxyde. On peut également incorporer un alcool aliphatique inférieur tel que de l'éthanol et éventuellement une huile de silicone, des glycols tels que les polypropylène- ou polyéthylène glycols.

Les formulations des compositions de l'invention peuvent être diverses et leur mise au point pour des besoins spécifiques appartient aux connaissances acquises par l'homme du métier.

Selon l'invention, la concentration du ou des principes actifs au sein de la composition peut varier assez largement en fonction, notamment, de la forme de présentation de la composition . En général, cependant, cette concentration se situe entre 0,1 et 15% en poids par rapport au poids total de la composition ,et en particulier entre 0,5 et 5% en poids. Des exemples illustratifs de compositions conformes à l'invention sont donnés ci-après .

L'acitivité du diazoxyde, de la phénytoïne ou de leurs sels se manifeste par une prévention de la chute des cheveux et une repousse lorsque le bulbe capillaire n'est pas atrophié. Les compositions selon l'invention sont donc utiles pour le traitement préventif ou curatif de la calvitie et des alopécies.

Les compositions de l'invention peuvent contenir une association de diazoxyde et de phénytoïne . Egalement, l'invention a pour objet des compositions dans lesquelles les agents actifs précités(diazoxyde et/ou phénytoïne)sont associés à une ou plusieurs substances capables de potentialiser leur effet. On citera ci-après des catégories de substances appropriées, avec l'indication (en poids , par rapport au total de la composition)de leur gamme de pourcentage pour obtenir l'effet de potentialisation désiré.

-Dérivés d'acides aminés, tels que l'acide octanoylcollagénique (lipoaminoacide caprylyl collagénique) , qui est le produit de condensa tion du chlorure d'octanoyle sur un hydrolysat alcalin de collagène, ou le "Lipacide $C_4HP$" (butyryl hydroxyproline),qui est obtenu par acylation de l'anhydride butyrique sur la fonction amine de l'hydroxyproline, ces dérivés étant utilisables dans la gamme de 0,1 à 5%.

-Dérivés de l'acide nicotinique, tels que nicotinate de benzyle: 0,1 à 5% (voir Merck Index , 10ème Edition p.935, nº 6367)

-Dérivés soufrés, tels que carbocystéine (voir Merck Index, 10ème Edition, p.249, nº1785), Lipoaminoacide caprylylcystinique : 0,1 à 5%

-Enoxolone: 0,2 à 5% (Voir Merck Index, 10ème Edition, p.518, nº 3545)

-Adjuvants favorisant la pénétration des principes actifs tels que : DMSO : 0,1 à 3%

-Dérivés terpéniques tels que Menthol: 0,005 à 0,5 %, Thymol: 0,005 à 0,5% (Voir Merck Index , 10ème Edition,p.1347, nº9246).

-Dérivés vitaminiques tels que: acide panthothénique : 0,02 à 1% (Voir Merck Index, 10éme edition, p. 1007, nº6877), vitamine E, B6....

L'invention sera encore illustrée, sans être aucunement limitée, par les exemples suivants, qui décrivent diverses formes de présentation des nouvelles compositions.

<u>EXEMPLE 1</u>

<u>Lotion anhydre</u>

phénytoïne 0,7 g
éthanol 95% 70,0 g
monopropylène glycol qs 100 g

<u>EXEMPLE 2</u>

<u>gel hydroalcoolique</u>

phénytoïne 0,5 g
Veegum R (gélifiant à base de silicate Al-Mg) 1,2 g
méthylhydroxy propyl-cellulose (20.000 cps) 1,6 g
éthanol 20,0 g

eau purifée qs 100 g

EXEMPLE 3

Crème émulsion huile dans l'eau
phénytoïne 2,0 g
mono- et di-palmitate de polyéthylène glycol 10,0 g
acide stéarique 3,0 g
glycérides saturés $C_{12}$ à $C_{18}$ polyoxyéthylénés 3,0 g
palmitate d'isopropyle 4,0 g
monopropylène glycol 3,0 g
conservateurs eau purifiée qs 100 g

EXEMPLE 4

Crème émulsion huile dans l'eau
phénytoïne 2,0 g
ester d'acides gras polyoxyéthyléné (HLB: 8,7) 6,0 g
alcool cétylique 2,0 g
acide stéarique 5,0 g
huile de paraffine 3,0 g
2-octyl dodécanol 6,0 g
diméthicone 200(silicone) 1,0 g
propylène glycol 10,0 g
conservateurs eau purifiée qs 100 g

EXEMPLE 5

Lotion anhydre
nicotinate de benzyle 0,2 g
phénytoïne 0,7 g
éthanol 95% 70,0 g
monopropylène glycol qs 100,0 g

EXEMPLE 6

Gel hydroalcoolique
phénytoïne 0,5 g
butyryl hydroxyproline 1,0 g
Veegum R (gélifiant à base de silicate Al-Mg) 1,2 g
méthyl hydroxypropyl cellulose (20 000 cps) 1,6 g
éthanol 20,0 g
eau purifiée qs 100,0 g

EXEMPLE 7

Crème émulsion huile dans eau
phénytoïne 2 g
lipoaminoacide caprylylcollagénique 2,0 g
mono di-palmitate de polyéthylène glycol 10,0 g
acide stéarique 3,0 g
glycérides saturés $C_{12}$ à $C_{18}$ polyoxyéthylènés 3,0 g
palmitate d'isopropyle 4,0 g
monopropylène glycol 3,0 g
conservateurs eau purifiée qs 100,0 g

EXEMPLE 8

Crème émulsion huile dans eau
phénytoïne 2 g
lipoaminoacide caprylylcystinique 2,0 g
enoxolone 1,0 g
esters d'acides gras polyoxyéthylènés (HLB 8,7) 6,0 g
alcool cétylique 2,0 g
acide stéarique 5,0 g
huile de paraffine 3,0 g
2-octyl dodécanol 6,0 g
diméthicone 200 (silicone) 1,0 g
propylène glycol 10,0 g
conservateurs eau purifiée qs 100,0 g

EXEMPLE 9

Gel alcoolique
phénytoïne de sodium 0,75g
DMSO 1,00 g
éthanol 70,00 g
méthyl cellulose 0,5 g
monopropylène glycol qs 100,00 g

EXEMPLE 10

Lotion anhydre
phénytoïne 1,5 g
menthol 0,05 g
thymol 0,05 g
éthanol 95% 70,00 g
propylène glycol qs 100,00 g

EXEMPLE 11

Lotion anhydre
diazoxyde 0,25 g
DMSO 1,00 g
éthanol 70,00 g
monopropylène glycol qs 100,00 g

EXEMPLE 12

Gel alcoolique
diazoxyde 0,25 g
éthanol 70,00 g
méthyl cellulose 0,50 g
acide pantothénique 0,10 g
monopropylène glycol qs 100,00 g

EXEMPLE 13

Crème émulsion huile dans eau
diazoxyde 1,00 g
enoxolone 1,00 g
ester d'acide gras polyoxyéthylèné 6,00 g
alcool cétylique 5,00 g
huile de paraffine 5,00 g
isostéarate d'isostéaryle 3,00 g
propylène glycol 5,00 g
conservateurs carbocystéine 1,50 g
eau purifiée qs 100,0 g

EXEMPLE 14:

Lotion alcoolique
phénytoïne 1,4 g
diazoxyde 0,1 g
lipacide C8CV 0,1 g
triéthanolamine 0,5 g
monopropylène glycol 22,3 g
éthanol 95% 70,0 g
eau déminéralisée 5,6 g

EXEMPLE 15:

Gel alcoolique

phénytoïne 1,0 g
diazoxyde 0,4 g
monopropylène glycol 20,0 g
ethanol 95% 77,6%
Klucel MF (hydroxypropyl-cellulose) 1,0 g

Les compositions des exemples 1 et 11 ont été respectivement utilisées pour le traitement de deux groupes de dix volontaires, des hommes de 30 à 40 ans, ayant une tendance marquée à la chute des cheveux, mais n'ayant pas encore atteint un stade de calvitie totale à la partie supérieure du cuir chevelu. Chaque volontaire a appliqué quotidiennement la composition sous forme de friction avec massage léger du cuir chevelu. La durée totale du traitement a été de deux mois. De huit à neuf hommes dans chaque groupe ont constaté après ce traitement une amélioration se traduisant par un net ralentissement de la chute des cheveux, qu'ils ont observé en comparant subjectivement le nombre de cheveux retenus par le peigne ou la brosse, ou tombés sur les vêtements, avant et après le traitement.

La lotion à 1,4 % de phénytoïne (exemple 14) a été utilisée en applications quotidiennes sur des alopécies androgéniques chez 11 malades adultes , dont deux femmes.

Aucun effet secondaire initial ou topique n'a été mis en évidence après, pour les plus anciens d'entre eux, dix mois de traitement.

Un effet anti-chute a été observé, ainsi que trois cas de repousse sous forme de duvet, apparu après au moins trois mois de traitement.

Il va sans dire que la fréquence des applications et la durée du traitement à l'aide des compositions de l'invention dépendront de la gravité de l'affection à prévoir ou à guérir. Il convient aussi de souligner que la repousse des cheveux n'est possible que si les bulbes capillaires ne sont pas atteints d'une manière irréversible .

On notera également que les produits actifs des compositions de l'invention ont déjà été proposés comme médicaments,mais les compositions pharmaceutiques correspondantes n'ont pas été présentées en vue de l'application topique.

L'invention concerne donc d'une façon générale l'application de la phénytoïne, du diazoxyde ou de leurs sels, à titre de produits pour le traitement de la calvitie et des alopécies. Elle a également pour objet un procédé pour l'obtention desdits produits lorsqu'ils sont destinés à un tel traitement.

**Revendications**

1. Compositions cosmétiques et pharmaceutiques présentées en vue de l'application topique et contenant à titre d'agent actif, l'un au moins des produits connus sous les Dénominations Communes Internationales (DCI) Phénytoin (ou Phénytoïne) et Diazoxide (ou Diazoxyde), ainsi que leurs sels acceptables du point de vue cosmétique ou pharmaceutique.

2. Compositions selon la revendication 1,caractérisées en ce qu'elles contiennent, à titre d'agent actif, au moins un sel alcalin ou alcalino-terreux de la phénytoïne, en particulier le sel de sodium ou de calcium de celle-ci.

3. Compositions selon l'une des revendications 1 ou 2, présentées sous forme de lotions, gels, émulsions, pommades ou autres formes convenant à l'application topique, avec les excipients usuels pour ce type d'application.

4. Composition selon l'une quelconque des revendications 1 à 3, caractérisées en ce que la concentration de l'agent actif se situe entre 0,1 et 15% en poids par rapport au poids total de la composition, et en particulier entre 0,5 et 5% en poids .

5. Compositions selon l'une quelconque des revendications 1 à 4, caractérisées en ce que les agents actifs, à savoir le diazoxyde et/ou la phénytoïne ou leurs sels sont associés à une ou plusieurs substances capables de potentialiser leur effet, choisies notamment parmi les catégories ci-après, en quantités respectives indiquées en pourcent en poids , par rapport au poids total de la composition:

-Dérivés d'acides aminés, tels que l'acide octanoylcollagénique (lipoaminoacide caprylyl collagénique ), qui est le produit de condensation du chlorure d'octanoyle sur un hydrolysat alcalin de collagène, ou le "Lipacide $C_4HP$" (butyryl hydroxyproline), qui est obtenu par acylation de l'anhydride butyrique sur la fonction amine de l'hydroxyproline , ces dérivés étant utilisables dans la gamme de 0,1 à 5%.
-Dérivés de l'acide nicotinique, tels que nicotinate de benzyle : 0,1 à 5%.
-Dérivés soufrés, tels que carbocystéine, Lipoaminoacide caprylylcystinique: 0,1 à 5%
-Enoxolone: 0,2 à 5%
-Adjuvants favorisant la pénétration des principes actifs tels que DMSO : 0,1 à 3%.
-Dérivés terpéniques tels que Menthol: 0,005 à 0,5%, Thymol: 0,005 à 0,5%.
-Dérivés vitaminiques tels que acide panthothénique : 0,02 à 1%, vitamine E, B6..

6.Compositions selon l'une quelconque des revendications 1 à 5,pour le traitement préventif ou curatif de la calvitie et des alopécies.

7.Application de la phénytoïne, du diazoxyde ou de leurs sels, à titre de produits pour le traitement de la calvitie et des alopécies.

8.Procédé pour l'obtention de phénytoïne, de diazoxyde ou de leurs sels, en tant que produits destinés au traitement de la calvitie et des alopécies.

Office européen des brevets

# RAPPORT DE RECHERCHE EUROPEENNE

Numero de la demande

EP 87 40 0868

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 027 655 (WELLA)<br>* Revendications * | 1,3,4, 5,6,7 | A 61 K 7/06 |
| X | GB-A-2 139 891 (L'OREAL)<br>* Revendications; page 1, lignes 29-32,50-54 * | 1,3,8 | |
| A | CHEMICAL ABSTRACTS, vol. 68, 1968, page 6979, résumé no. 72215a, Columbus, Ohio, US; & CS-A-123 217 (KAREL FISER et al.) 15-06-1967<br>* Abrégé * | 1 | |
| A | CHEMICAL ABSTRACTS, vol. 52, 1958, page 17, résumé no. 4101d,Columbus, Ohio, US; S. COHEN: "Dimethylhydantoin derivatives", & DRUG & COSMETIC IND. 81, 306-7, 388-9, 392-3(1957)<br>* Abrégé * | 1 | DOMAINES TECHNIQUES RECHERCHES (Int. Cl.4)<br>A 61 K |
| D,X | THE MERCK INDEX, an encyclopedia of chemicals and drugs, édition 9, pages 395-952, 1976, Merck & Co., Inc., Rahway, N.J., US<br>* Page 395, no. 2968; page 952, no. 7130 * | 8 | |

Le présent rapport de recherche a été établi pour toutes les revendications

| Lieu de la recherche | Date d'achèvement de la recherche | Examinateur |
|---|---|---|
| LA HAYE | 05-08-1987 | WILLEKENS G.E.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82